# EUROPEAN PATENT SPECIFICATION

(11) **EP 2 277 999 B1**
(45) Date of publication and mention of the grant of the patent: **02.07.2014**
(21) Application number: 10180769.1
(22) Date of filing: 30.03.2000
(51) Int. Cl.: C12N 9/20, C11D 3/386, C12N 5/10

(54) **Lipase variant**
Lipasevariante
Variante de lipase

(30) Priority: 31.03.1999 DK 44199
(43) Date of publication of application: 26.01.2011
(62) Divisional of application: 03024829.8
(73) Proprietor: Novozymes A/S, 2880 Bagsvaerd (DK)
(72) Inventor: Vind, Jesper, 3500, Vaerloese (DK); Svendsen, Allan, 2970, Hoersholm (DK); Patkar, Shamkant Anant, 2800, Kgs. Lyngby (DK); Andersen, Kim V, 2700, Bronshoej (DK); Halkier Dorte A, 2680, Solroed Strand (DK); Bojsen, Kirsten, 2900, Hellerup (DK)

(56) References cited:
- WO-A-97/07202
- WO-A-99/42566
- US-A- 5 869 438

## Description

### FIELD OF THE INVENTION

The present invention relates to lipase variants suited for use in detergent compositions. More particularly, the invention relates to variants of the wild-type lipase from *Humicola lanuginosa* strain DSM 4109 showing a first-wash effect.

### BACKGROUND OF THE INVENTION

For a number of years, lipases have been used as detergent enzymes to remove lipid or fatty stains from clothes and other textiles, particularly a lipase derived from *Humicola lanuginosa* (EP258068 and EP305216) sold under the tradename Lipolase^{®} (product of Novo Nordisk A/S).

WO92/05249, WO94/25577, WO95/22615, WO97/04079 and WO97/07202 disclose variants of the *H. lanuginosa* lipase having improved properties for detergent purposes. Thus, WO97/04079 discloses variants having a peptide addition (extension) at the N-terminal and/or the C-terminal. WO97/07202 discloses lipase variants with "first wash performance" which are capable of removing substantial amounts of lard from a lard stained swatch in a one-cycle wash.

There is an ever existing need for providing novel lipases with improved washing properties in a variety of commercial detergents. The present invention relates to such novel lipases.

### SUMMARY OF THE INVENTION

The inventors have found that certain variants of Lipolase (wild-type *Humicola lanuginosa* lipase) have a particularly good first-wash performance in a detergent solution. The lipases may further provide additional benefits, such as whiteness maintenance and dingy cleanup.

The inventors found that the variants should comprise one or more substitutions with positive amino acids near the N-terminal in the three-dimensional structure. The variants should further comprise a peptide addition at the C-terminal and/or should meet certain limitations on electrically charged amino acids at positions 90-101 and 210.

Accordingly, the invention provides a lipase which is a polypeptide having an amino acid sequence which:
a) has at least 90% identity with the wild-type lipase derived from *Humicola lanuginosa* strain DSM 4109;
b) compared to said wild-type lipase, comprises a substitution T231 R; and
   i) comprises a negative amino acid in position E210 of said wild-type lipase;
   ii) comprises a negatively charged amino acid in the region corresponding to positions 90-101 of said wild-type lipase; and
   iii) comprises a neutral or negative amino acid at a position corresponding to N94 of said wild-type lipase and/or has a negative or neutral net electric charge in the region corresponding to positions 90-101 of said wild-type lipase.

### DETAILED DESCRIPTION OF THE INVENTION

### Humicola lanuginosa lipase

The reference lipase used in this invention is the wild-type lipase derived from *Humicola lanuginosa* strain DSM 4109. It is described in EP258068 and EP305216 and has the amino acid sequence shown in positions 1-269 of SEQ ID NO: 2 of US5869438. In this specification, the reference lipase is also referred to as Lipolase.

### Substitution with positive amino acid

The lipase of the invention comprises one or more (e.g. 2-4, particularly two) substitutions of an electrically neutral or negatively charged amino acid near E1 or Q249 with a positively charged amino acid, preferably R.

The substitution is at the surface of the three-dimensional structure within 15Å of E1 or Q249, e.g. at any of positions 1-11, 90, 95, 169, 171-175, 192-211, 213-226, 228-258, 260-262.

The substitution may be within 10Å of E1 or Q249, e.g. at any of positions 1-7, 10, 175, 195, 197-202, 204-206, 209, 215, 219-224, 230-239, 242-254.

The substitution may be within 15Å of E1, e.g. at any of positions 1-11, 169, 171, 192-199, 217-225, 228-240, 243-247, 249, 261-262.

The substitution is most preferably within 10Å of E1, e.g. at any of positions 1-7, 10, 219-224 and 230-239.

Thus, some preferred substitutions are S3R, S224R, P229R, T231 R, N233R, D234R and T244R.

### Peptide addition at C-terminal

The lipase may comprise a peptide addition attached to C-terminal L269. The peptide addition improves the first-wash performance in a variety of detergents.

The peptide addition preferably consists of 1-5 amino acids, e.g. 2, 3 or 4 amino acids. The amino acids of the peptide addition will be numbered 270, 271, etc.

The peptide addition may consist of electrically neutral (e.g. hydrophobic) amino acids, e.g. PGL or PG. In an alternative embodiment, the lipase peptide addition consists of neutral (e.g. hydrophobic) amino acids and the amino acid C, and the lipase comprises substitution of an amino acid with C at a suitable location so as to form a disulfide bridge with the C of the peptide addition. Examples are:
270C linked to G23C or T37C
271C linked to K24C, T37C, N26C or R81C
272C linked to D27C, T35C, E56C, T64C or R81C.

### Amino acids at positions 90-101 and 210

The lipase of the invention preferably meets certain limitations on electrically charged amino acids at positions 90-101 and 210. Lipases meeting the charge limitations are particularly effective in a detergent with high content of anionic.

Thus, amino acid 210 may be negative. E210 may be unchanged or it may have the substitution E210D/C/Y, particularly E210D.

The lipase may comprise a negatively charged amino acid at any of positions 90-101 (particularly 94-101), e.g. at position D96 and/or E99.

Further, the lipase may comprise a neutral or negative amino acid at position N94, i.e. N94(neutral or negative), e.g. N94N/D/E.

Also, the lipase may have a negative or neutral net electric charge in the region 90-101 (particularly 94-101), i.e. the number of negative amino acids is equal to or greater than the number of positive amino acids. Thus, the region may be unchanged from Lipolase, having two negative amino acids (D96 and E99) and one positive (K98), and having a neutral amino acid at position 94 (N94), or the region may be modified by one or more substitutions.

Alternatively, two of the three amino acids N94, N96 and E99 may have a negative or unchanged electric charge. Thus, all three amino acids may be unchanged or may be changed by a conservative or negative substitution, i.e. N94 (neutral or negative), D(negative) and E99(negative). Examples are N94D/E and D96E. Also, one of the three may be substituted so as to increase the electric charge, i.e. N94 (positive), D96 (neutral or positive) or E99 (neutral or positive). Examples are N94K/R, 96I/L/N/S/W or E99N/Q/K/R/H.

The substitution of a neutral with a negative amino acid (N94D/E), may improve the performance in an anionic detergent. The substitution of a neutral amino acid with a positive amino acid (N94K/R) may provide a variant lipase with good performance both in an anionic detergent and in an anionic/non-ionic detergent (a detergent with e.g. 40-70% anionic out of total surfactant).

### Amino acids at other positions

The inventors have found that a substitution Q249R/K/H may improve the performance both in anionic and in anionic/non-ionic detergent, and that a substitution of R209 with a neutral or negative amino acid (e.g. R209P/S) may improve the performance in anionic detergent. The lipase may optionally comprise the substitution G91A.

The lipase may optionally comprise substitutions of one or more additional amino acids. Such substitutions may, e.g., be made according to principles known in the art, e.g. substitutions described in WO92/05249, WO94/25577, WO95/22615, WO97/04079 and WO97/07202.

### Combinations of substitutions

A lipase variant with good first-wash performance may be obtained by modifying Lipolase as follows. Substitutions in parentheses are optional.

| |
|---|
| T231R+ N233R |
| N94K+ D96L+ T231R+ N233R+ Q249R+ 270P+ 271G+ 272L |
| D96L+ T231R+ N233R |
| G91A+ E99K+ T231R+ N233R+ Q249R |
| (N33Q) +D96L +T231R +N233R +Q249R +270 PGL |
| R209P +T231R +N233R |
| (N33Q) +E99N +N101S +T231R +N233R +Q249R +270 PGL |
| K24C +(N33Q) +D96S +T231R +N233R +Q249R +270 PCL |
| (N33Q) +G91A +E99K +T231R +N233R +Q249R +270 PGL |
| E1A +(N33Q) +G91A +E99K +T231R +N233R +Q249R +270 PGL |
| (N33Q) +G91A +E99K +G255R +T231R +N233R +Q249R +270 PGL |
| (N33Q) +G91A +E99K +T231R +N233R +T244R +Q249R +270 PGL |
| G91A +E99K +T231R +N233R +Q249R |
| E87K +G91D +D96L +G225P +T231R +N233R +Q249R +N251D |
| G91A +E99K +T231R +N233R +Q249R +270AGVF |
| G91A +E99K +T189G +T231R +N233R +Q249R |
| D102G +T231R +N233R +Q249R |
| T231R +N233R +Q249R +270AGVF |
| R209P +T231R +N233R |
| N33Q +N94K +D96L +T231R +N233R +Q249R +270PGLPFKRV |
| N33Q +N94K +D96L +T231R +N233R +Q249R |
| N33Q +D96S +T231R +N233R +Q249R |
| N33Q +D96S +V2281 + +T231R +N233R +Q249R |
| E1A +N33Q +G91A +E99K +T231R +N233R +Q249R +270PGLPFKRV |
| N33Q +S83T +E87K +G91A + E99K +T231R +N233R +Q249R +270PGLPFKRV |
| N33Q +G91A + E99K +T231R +N233R +Q249R +270PGLPFKRV |
| T231R +N233R +270CP |
| T231R +N233R +270RE |
| N33Q +E99N +N101S +T231R +N233R +Q249R +270PGLPFKRV |
| D62A +S83T + G91A +E99K +T231R +N233R +Q249R |
| E99N +N101S +T231R +N233R +Q249R |
| R84W +G91A +E99K +T231R +N233R +Q249R |
| G91A +E99K +T231R +N233R +Q249R +270SPG |
| G91A +E99K +T231R +N233R +Q249R +270VVVP |
| G91A +E99K +T231R +N233R +Q249R +270LLASSGRGGHR |
| G91A +E99K +T231R +N233R +Q249R +270VTT |
| G91A +E99K +T231R +N233R +Q249R +270VLQ |
| G91A +E99K +T231R +N233R +Q249R +270TST |
| G91A +E99K +T231R +N233R +Q249R +270LRI |
| V60G +D62E +G91A +E99K +T231R +N233R +Q249R |
| G91A +D96W +E99K +T231R +N233R +G263Q +L264A +I265T +G266S +T267A +L269N +270AGGFS |

### Nomenclature for amino acid modifications

The nomenclature used herein for defining mutations is essentially as described in WO92/05249. Thus, T231 R indicates a substitution of T in position 231 with R. PGL or 270P+271 G+272L indicates the peptide addition PGL attached to the C-terminal (L269).

### Amino acid grouping

In this specification, amino acids are classified as negatively charged, positively charged or electrically neutral according to their electric charge at pH 10, which is typical of the detergent of the invention. Thus, negative amino acids are E, D, C (cysteine) and Y, particularly E and D. Positive amino acids are R, K and H, particularly R and K. Neutral amino acids are G, A, V, L, I, P, F, W, S, T, M, N, Q and C when forming part of a disulfide bridge. A substitution with another amino acid in the same group (negative, positive or neutral) is termed a conservative substitution.

The neutral amino acids may be divided into hydrophobic (G, A, V, L, I, P, F, W and C as part of a disulfide bridge) and hydrophilic (S, T, M, N, Q).

### Amino acid identity

The lipase variant of the invention has an amino acid identity of at least 90% (preferably more than 95% or more than 98%) with Lipolase.

The degree of identity may be suitably determined by means of computer programs known in the art, such as GAP provided in the GCG program package (Program Manual for the Wisconsin Package, Version 8, August 1994, Genetics Computer Group, 575 Science Drive, Madison, Wisconsin, USA 53711) (Needleman, S.B. and Wunsch, C.D., (1970), Journal of Molecular Biology, 48, 443-45), using GAP with the following settings for polypeptide sequence comparison: GAP creation penalty of 3.0 and GAP extension penalty of 0.1.

### DNA sequence, Expression vector, Host cell, Production of lipase

The invention provides a DNA sequence encoding the lipase of the invention, an expression vector harboring the DNA sequence, and a transformed host cell containing the DNA sequence or the expression vector. These may be obtained by methods known in the art.

The invention also provides a method of producing the lipase by culturing the transformed host cell under conditions conducive for the production of the lipase and recovering the lipase from the resulting broth. The method may be practiced according to principles known in the art.

### Detergent additive

According to the invention, the lipase may typically be used as an additive in a detergent composition. This additive is conveniently formulated as a non-dusting granulate, a stabilized liquid, a slurry or a protected enzyme. The additive may be prepared by methods known in the art.

### DETERGENT COMPOSITION

The detergent compositions of the invention may for example, be formulated as hand and machine laundry detergent compositions including laundry additive compositions and compositions suitable for use in the pretreatment of stained fabrics, rinse added fabric softener compositions, and compositions for use in general household hard surface cleaning operations and dishwashing operations.

The detergent composition of the invention comprises the lipase of the invention and a surfactant. Additionally, it may optionally comprise a builder, another enzyme, a suds suppresser, a softening agent, a dye-transfer inhibiting agent and other components conventionally used in detergents such as soil-suspending agents, soil-releasing agents, optical brighteners, abrasives, bactericides, tarnish inhibitors, coloring agents, and/or encapsulated or non-encapsulated perfumes.

The detergent composition according to the invention can be in liquid, paste, gels, bars or granular forms. The pH (measured in aqueous solution at use concentration) will usually be neutral or alkaline, e.g. in the range of 7-11, particularly 9-11. Granular compositions according to the present invention can also be in "compact form", i.e. they may have a relatively higher density than conventional granular detergents, i.e. form 550 to 950 g/l.

The lipase of the invention, or optionally another enzyme incorporated in the detergent composition, is normally incorporated in the detergent composition at a level from 0.00001% to 2% of enzyme protein by weight of the composition, preferably at a level from 0.0001 % to 1% of enzyme protein by weight of the composition, more preferably at a level from 0.001% to 0.5% of enzyme protein by weight of the composition, even more preferably at a level from 0.01% to 0.2% of enzyme protein by weight of the composition.

The detergent composition of the invention may comprise the lipase in an amount corresponding to 10-50,000 LU per gram of detergent, preferably 20-5,000 LU/g, e.g. 100-1000 LU/g. The detergent may be dissolved in water to produce a wash liquor containing lipolytic enzyme in an amount corresponding to 25-15,000 LU per liter of wash liquor, particularly 100 - 5000 LU/I, e.g. 300-2000 LU/I. The amount of lipase protein may be 0.001-10 mg per gram of detergent or 0.001-100 mg per liter of wash liquor.

More specifically, the lipase of the invention may be incorporated in the detergent compositions described in WO97/04079, WO97/07202, WO97/41212, PCT/DK WO98/08939 and WO97/43375.

### Surfactant system

The surfactant system may comprise nonionic, anionic, cationic, ampholytic, and/or zwitterionic surfactants. As described above, the lipase variants of the invention are particularly suited for detergents comprising of a combination of anionic and nonionic surfactant with 70-100% by weight of anionic surfactant and 0-30% by weight of nonionic, particularly 80-100% of anionic surfactant and 0-20% nonionic. As further described, some preferred lipases of the invention are also suited for detergents comprising 40-70% anionic and 30-60% non-ionic surfactant.

The surfactant is typically present at a level from 0.1% to 60% by weight, e.g. 1% to 40%, particularly 10-40 %. preferably from about 3% to about 20% by weight. Some examples of surfactants are described below.

### Anionic surfactants

Preferred anionic surfactants include alkyl sulfate, alkyl ethoxy sulfate, linear alkyl benzene sulfonate and mixtures of these.

The alkyl sulfate surfactants are water soluble salts or acids of the formula ROSO₃M wherein R preferably is a C₁₀-C₂₄ hydrocarbyl, preferably an alkyl or hydroxyalkyl having a C₁₀-C₂₀ alkyl component, more preferably a C₁₂-C₁₈ alkyl or hydroxyalkyl, and M is H or a cation, e.g., an alkali metal cation (e.g. sodium, potassium, lithium), or ammonium or substituted ammonium.

Alkylbenzene sulfonates are suitable, especially linear (straight-chain) alkyl benzene sulfonates (LAS) wherein the alkyl group preferably contains from 10 to 18 carbon atoms.

Suitable anionic surfactants include alkyl alkoxylated sulfates which are water soluble salts or acids of the formula RO(A)ₘSO₃M wherein R is an unsubstituted C₁₀-C-₂₄ alkyl or hydroxyalkyl group having a 6₁₀-C₂₄ alkyl component, preferably a C₁₂-C₂₀ alkyl or hydroxyalkyl, more preferably C₁₂-C₁₈ alkyl or hydroxyalkyl, A is an ethoxy or propoxy unit, m is greater than zero, typically between about 0.5 and about 6, more preferably between about 0.5 and about 3, and M is H or a cation which can be, for example, a metal cation (e.g., sodium, potassium, lithium, calcium, magnesium, etc.), ammonium or substituted-ammonium cation. Alkyl ethoxylated sulfates as well as alkyl propoxylated sulfates are contemplated herein. Specific examples of substituted ammonium cations include methyl-, dimethyl, trimethyl-ammonium cations and quaternary ammonium cations such as tetramethylammonium and dimethyl piperdinium cations and those derived from alkylamines such as ethylamine, diethylamine, triethylamine, mixtures thereof, and the like.

Other anionic surfactants include salts (including, for example, sodium, potassium, ammonium, and substituted ammonium salts such as mono- di- and triethanolamine salts) of soap, C₈-C₂₂ primary or secondary alkanesulfonates, C₈-C₂₄ olefinsulfonates, sulfonated polycarboxylic acids prepared by sulfonation of the pyrolyzed product of alkaline earth metal citrates.

### Nonionic surfactant

The surfactant may comprise polyalkylene oxide (e.g. polyethylene oxide) condensates of alkyl phenols. The alkyl group may contain from about 6 to about 14 carbon atoms, in a straight chain or branched-chain. The ethylene oxide may be present in an amount equal to from about 2 to about 25 moles per mole of alkyl phenol.

The surfactant may also comprise condensation products of primary and secondary aliphatic alcohols with about 1 to about 25 moles of ethylene oxide. The alkyl chain of the aliphatic alcohol can either be straight or branched, and generally contains from about 8 to about 22 carbon atoms.

Further, the nonionic surfactant may comprise polyethylene oxide condensates of alkyl phenols, condensation products of primary and secondary aliphatic alcohols with from about 1 to about 25 moles of ethylene oxide, alkylpolysaccharides, and mixtures hereof. Most preferred are C₈-C₁₄ alkyl phenol ethoxylates having from 3 to 15 ethoxy groups and C₈-C₁₈ alcohol ethoxylates (preferably C₁₀ avg.) having from 2 to 10 ethoxy groups, and mixtures thereof.

Preferred nonionic surfactants are alcohol ethoxylate, alcohol phenol ethoxylate, polyhydroxy fatty acid amide, alkyl polyglucoside and mixtures of these.

### EXAMPLE

### First-wash performance at various dosages in anionic detergent

The following 5 variants according to the invention were prepared and tested with 3 types of soiled swatches, and the parent lipase was included for comparison:

| |
|---|
| T231R +N233R. |
| G91A +D96W +E99K +G263Q +L264A +I265T +G266D +T267A +L269N +270AGGFSWRRYRSAESVDKRATMTDAELEKKLNSYVQMDKEYVKNNQARS |
| R209P +T231R +N233R |
| N33Q +D96S +T231R +N233R +Q249R |
| E99N +N101S +T231R +N233R +Q249R |

A commercial US detergent with a high content of anionic surfactant was heated to inactivate the enzymes already present, and was used at 1.4 g/l in a Tergot-o-meter^{™} laboratory washing machine. The water hardness was 6°dH (Ca:Mg 2:1), and the washing conditions were 1 cycle at 30°C for 12 minutes, followed by over-night drying on filter paper. The lipase variant was added at dosages of 6400 and 12800 LU/I. Double determinations were made in two separate washes (same TOM).

The following three types of textile swatches tested were: Lard/Sudan Red on cotton Style 400, freshly prepared; wfk 20-LS Lipstick on polyester/cotton; and wfk 10-LS lipstick on cotton. The soil removal was evaluated by measuring the remission at 460 nm after the first washing cycle, and the results were expressed as ΔR by subtracting the remission of a blank without enzyme.

The results were that each variant at each dosage showed an improved first-wash performance on each type of soiled swatch (ΔR of 5 to 13), whereas the parent enzyme had essentially no effect (ΔR = 0 ± 2) under the same conditions.

## Claims

1. A lipase which is a polypeptide having an amino acid sequence which:
a) has at least 90 % identity with the wild-type lipase derived from *Humicola lanuginosa* strain DSM 4109;
b) compared to said wild-type lipase, comprises a substitution T231R; and
i) comprises a negative amino acid in position E210 of said wild-type lipase;
ii) comprises a negatively charged amino acid in the region corresponding to positions 90-101 of said wild-type lipase; and
iii) comprises a neutral or negative amino acid at a position corresponding to N94 of said wild-type lipase and/or has a negative or neutral net electric charge in the region corresponding to positions 90-101 of said wild-type lipase.

2. The lipase of claim 1 which comprises a substitution of an electrically neutral or negatively charged amino acid at any of positions 1-7, 10, 219-224 and 230-239 with R.

3. The lipase of claim 1 or 2 which comprises a substitution of amino acid S3, S224, P229, N233, D234 or T244 with a positively charged amino acid, preferably R.

4. The lipase of any preceding claim which comprises a peptide addition at the C-terminal.

5. The lipase of claim 4 wherein the peptide addition consists of 1-5 amino acids.

6. The lipase of claim 4 or 5 wherein the peptide addition consists of electrically neutral (preferably hydrophobic) amino acids, and is most preferably PGL or PG.

7. The lipase of any of claims 4-6 wherein the peptide addition consists of neutral (preferably hydrophobic) amino acids and C, and the lipase further comprises substitution of an amino acid with C so as to form a disulfide bridge with the C of the peptide addition.

8. The lipase of any preceding claim which comprises amino acids with negative or unchanged electric charge in at least two of positions N94, D96 and E99 of said wild-type lipase.

9. The lipase of any preceding claim which comprises a substitution G91A, N94D/E/K/R, D96E/I/L/N/S/W, E99N/Q/K/R/H, N101S, R209P/S or Q249R/K/H.

10. The lipase of any preceding claim which comprises one of the following sets of substitutions, optionally combined with Q249R/K/H and/or K98X:
a) G91 G/A/S/T + N94(neutral or negative) + D96D/E/C/Y + E99E/D/C/Y,
b) G91G/A/S/T + N94(neutral or negative) + D96D/E/C/Y + E99N + N101 S,
c) G91 G/A/S/T + N94R/K/H + D96D/E/C/Y + E99E/D/C/Y,
d) G91G/A/S/T + N94(neutral or negative) + D96(neutral or positive) + E99E/D/C/Y, or
e) G91G/A/S/T + N94(neutral or negative) + D96D/E/C/Y + E99(neutral or positive).

11. The lipase of any of claims 1-10 which comprises one of the following sets of substitutions, optionally combined with R209(neutral or negative):
a) E99R/K/H + Q249R/K/H,
b) N94R/K/H + Q249R/K/H, or
c) D96(neutral or positive) + Q249R/K/H.

12. The lipase of any of claims 1-11 which comprises one of the following sets of substitutions:
a) G91A +E99K +T231 R +N233R +Q249R;
b) E99N +N101S +T231 R +N233R +Q249R +270PGL;
c) N33Q +E99N +N101S +T231R +N233R +Q249R +270PGL;
d) G91A +E99K +T231 R +N233R +Q249R +270PGL;
e) N33Q +G91A +E99K +T231R +N233R +Q249R +270PGL;
f) E1A +G91A +E99K +T231 R +N233R +Q249R +270PGL;
g) E1A +N33Q +G91A +E99K +T231 R +N233R +Q249R +270PGL;
h) G91A +E99K +G255R +T231 R +N233R +Q249R +270PGL;
i) N33Q +G91A +E99K +G255R +T231 R +N233R +Q249R +270PGL;
j) G91A +E99K +T231 R +N233R +T244R +Q249R +270PGL;
k) N33Q +G91A +E99K +T231 R +N233R +T244R +Q249R +270PGL;
l) G91A +E99K +T231 R +N233R +Q249R;
m) G91A +E99K +T231 R +N233R +Q249R +270AGVF;
n) G91A +E99K +T189G +T231 R +N233R +Q249R;
o) E1A +N33Q +G91A +E99K +T231R +N233R +Q249R +270PGLPFKRV;
p) N33Q +S83T +E87K +G91A + E99K +T231 R +N233R +Q249R +270PGLPFKRV;
q) N33Q +G91A + E99K +T231 R +N233R +Q249R +270PGLPFKRV;
r) N33Q +E99N +N101 S +T231 R +N233R +Q249R +270PGLPFKRV;
s) D62A +S83T + G91A +E99K +T231 R +N233R +Q249R;
t) E99N +N101S +T231 R +N233R +Q249R;
u) R84W +G91A +E99K +T231 R +N233R +Q249R;
v) G91A +E99K +T231 R +N233R +Q249R +270SPG;
w) G91A +E99K +T231 R +N233R +Q249R +270VVVP;
x) G91A +E99K +T231 R +N233R +Q249R +270LLASSGRGGHR;
y) G91A +E99K +T231 R +N233R +Q249R +270VTT;
z) G91A +E99K +T231 R +N233R +Q249R +270VLQ;
aa) G91A +E99K +T231 R +N233R +Q249R +270TST;
bb) G91A +E99K +T231 R +N233R +Q249R +270LRI;
cc) V60G +D62E +G91A +E99K +T231 R +N233R +Q249R;
dd) G91A +D96W +E99K +T231 R +N233R +G263Q +L264A +I265T +G266S +T267A +L269N +270AGGFS;
ee) G91A +D96W +E99K +G263Q +L264A +I265T +G266D +T267A +L269N +270AGGFSWRRYRSAESVDKRATMTDAELEKKLNSYVQMDKEYVKNNQAR S.

13. A detergent composition comprising a surfactant and the lipase of any of claims 1-12.

14. The detergent composition of the preceding claim wherein the surfactant comprises anionic surfactant in an amount of more than 70 % by weight of the total surfactant.

15. The detergent composition of claim 13, wherein the surfactant comprises anionic surfactant in an amount of 40-70 % by weight and nonionic surfactant in an amount of 30-60 % by weight of the total surfactant, and the lipase is the lipase of claim 6 or 7.

16. The detergent composition of any of claims 13-15 which comprises 10-40 % by weight surfactant, preferably comprises 40-70 % builder, and preferably has a pH of 9-11 when dissolved in water at 0.5-5 g/l.

17. A DNA sequence encoding the lipase of any of claims 1-12.

18. An expression vector harboring the DNA sequence of the preceding claim.

19. A transformed host cell containing the DNA sequence of claim 17 or the expression vector of claim 18.

20. A method of producing the lipase of any of claims 1-12 which method comprises culturing the transformed host cell of claim 19 under conditions conducive for the production of the lipase and recovering the lipase from the resulting broth.

## Patentansprüche

1. Lipase, die ein Polypeptid mit einer Aminosäuresequenz ist, die:
a) mindestens 90 % Identität mit der aus *Humicola lanuginosa* Stamm DSM 4109 abgeleiteten Wildtyp-Lipase aufweist;
b) verglichen mit der Wildtyp-Lipase eine Substitution T231 R umfasst; und
i) eine negative Aminosäure in Position E210 der Wildtyp-Lipase umfasst;
ii) eine negativ geladene Aminosäure in der Region entsprechend Positionen 90-101 der Wildtype-Lipase umfasst; und
iii) eine neutrale or negative Aminosäure an einer Position entsprechend N94 der Wildtyp-Lipase umfasst und/oder eine negative or neutrale elektrische Nettoladung in der Region entsprechend Positionen 90-101 der Wildtyp-Lipase aufweist.

2. Lipase nach Anspruch 1, die eine Substitution einer elektrisch neutralen or negativ geladenen Aminosäure an einer beliebigen der Positionen 1-7, 10, 219-224 und 230-239 mit R aufweist.

3. Lipase nach Anspruch 1 oder 2, die eine Substitution von Aminosäure S3, S224, P229, N233, D234 oder T244 mit einer positiv geladenen Aminosäure, bevorzugt R, umfasst.

4. Lipase nach einem beliebigen voranstehenden Anspruch, die eine Peptidaddition am C-Terminus umfasst.

5. Lipase nach Anspruch 4, wobei die Peptidaddition aus 1-5 Aminosäuren besteht.

6. Lipase nach Anspruch 4 oder 5, wobei die Peptidaddition aus elektrisch neutralen (bevorzugt hydrophoben) Aminosäuren besteht, und am meisten bevorzugt PGL oder PG ist.

7. Lipase nach einem beliebigen der Ansprüche 4-6, wobei die Peptidaddition aus neutralen (bevorzugt hydrophoben) Aminosäuren und C besteht, und die Lipase weiterhin Substitution einer Aminosäure mit C umfasst, um eine Disulfidbrücke mit dem C der Peptidaddition zu bilden.

8. Lipase nach einem beliebigen der voranstehenden Ansprüche, die Aminosäuren mit negativer oder unveränderter elektrischer Ladung in mindestens zwei der Positionen N94, D96 und E99 der Wildtyp-Lipase umfasst.

9. Lipase nach einem beliebigen der voranstehenden Ansprüche, die eine Substitution G91A, N94D/E/K/R, D96E/I/L/N/S/W, E99N/Q/K/R/H, N101S, R209P/S oder Q249R/K/H umfasst.

10. Lipase nach einem beliebigen der voranstehenden Ansprüche, die einen der folgenden Sätze an Substitutionen umfasst, gegebenenfalls kombiniert mit Q249R/K/H und/oder K98X:
a) G91 G/A/S/T + N94(neutral oder negativ) + D96D/E/C/Y + E99E/D/C/Y,
b) G91 G/A/S/T + N94(neutral oder negativ) + D96D/E/C/Y + E99N + N101S,
c) G91G/A/S/T + N94R/K/H + D96D/E/C/Y + E99E/D/C/Y,
d) G91 G/A/S/T + N94(neutral oder negativ) + D96(neutral oder positiv) + E99E/D/C/Y, oder
e) G91 G/A/S/T + N94(neutral oder negativ) + D96D/E/C/Y + E99(neutral oder positiv).

11. Lipase nach einem beliebigen der Ansprüche 1-10, die einen der folgenden Sätze an Substitutionen umfasst, gegebenenfalls kombiniert mit R209(neutral oder negativ):
a) E99R/K/H + Q249R/K/H,
b) N94R/K/H + Q249R/K/H, oder
c) D96(neutral oder positiv) + Q249R/K/H.

12. Lipase nach einem beliebigen der Ansprüche 1-11, die einen der folgenden Sätze an Substitutionen umfasst:
a) G91A +E99K +T231 R +N233R +Q249R;
b) E99N +N101S +T231 R +N233R +Q249R +270PGL;
c) N33Q +E99N +N101S +T231R +N233R +Q249R +270PGL;
d) G91A +E99K +T231 R +N233R +Q249R +270PGL;
e) N33Q +G91A +E99K +T231 R +N233R +Q249R +270PGL;
f) E1A+G91A+E99K +T231 R +N233R +Q249R +270PGL;
g) E1A+N33Q +G91A +E99K +T231R +N233R +Q249R +270PGL;
h) G91A +E99K +G255R +T231 R +N233R +Q249R +270PGL;
i) N33Q +G91A +E99K +G255R +T231 R +N233R +Q249R +270PGL;
j) G91A +E99K +T231 R +N233R +T244R +Q249R +270PGL;
k) N33Q +G91A +E99K +T231 R +N233R +T244R +Q249R +270PGL;
l) G91A +E99K +T231 R +N233R +Q249R;
m) G91A +E99K +T231 R +N233R +Q249R +270AGVF;
n) G91A +E99K +T189G +T231 R +N233R +Q249R;
o) E1A +N33Q +G91A +E99K +T231 R +N233R +Q249R +270PGLPFKRV;
p) N33Q +S83T +E87K +G91A + E99K +T231R +N233R +Q249R +270PGLPFKRV;
q) N33Q +G91A + E99K +T231R +N233R +Q249R +270PGLPFKRV;
r) N33Q +E99N +N101S +T231R +N233R +Q249R +270PGLPFKRV;
s) D62A +S83T + G91A +E99K +T231R +N233R +Q249R;
t) E99N +N101S +T231R +N233R +Q249R;
u) R84W +G91A +E99K +T231R +N233R +Q249R;
v) G91A +E99K +T231R +N233R +Q249R +270SPG;
w) G91A +E99K +T231R +N233R +Q249R +270VVVP;
x) G91A +E99K +T231R +N233R +Q249R +270LLASSGRGGHR;
y) G91A +E99K +T231R +N233R +Q249R +270VTT;
z) G91A +E99K +T231R +N233R +Q249R +270VLQ;
aa) G91A +E99K +T231R +N233R +Q249R +270TST;
bb) G91A +E99K +T231R +N233R +Q249R +270LR1;
cc) V60G +D62E +G91A +E99K +T231R +N233R +Q249R;
dd) G91A +D96W +E99K +T231R +N233R +G263Q +L264A +I265T +G266S +T267A +L269N +270AGGFS;
ee) G91A +D96W +E99K +G263Q +L264A +I265T +G266D +T267A +L269N +270AGGFSWRRYRSAESVDKRATMTDAELEKKLNSYVQMDKEYVKNNQARS.

13. Detergenszusammensetzung umfassend ein oberflächenaktives Mittel und die Lipase eines beliebigen der Ansprüche 1-12.

14. Detergenszusammensetzung des voranstehenden Anspruchs, wobei das oberflächenaktive Mittel anionisches oberflächenaktives Mittel in einer Menge von mehr als 70 Gew.-% des gesamten oberflächenaktiven Mittels umfasst.

15. Detergenszusammensetzung nach Anspruch 13, wobei das oberflächenaktive Mittel anionisches oberflächenaktives Mittel in einer Menge von 40-70 Gew.-% und nichtionisches oberflächenaktives Mittel in einer Menge von 30-60 Gew.-% des gesamten oberflächenaktiven Mittels umfasst, und die Lipase die Lipase von Anspruch 6 oder 7 ist.

16. Detergenszusammensetzung nach einem beliebigen der Ansprüche 13-15, die 10-40 Gew.-% oberflächenaktives Mittel umfasst, bevorzugt 40-70 Gew.-% Builder umfasst, und bevorzugt einen pH von 9-11 aufweist, wenn sie in Wasser zu 0,5-5 g/l gelöst ist.

17. DNA-Sequenz, die die Lipase eines beliebigen der Ansprüche 1-12 kodiert.

18. Expressionsvektor, der die DNA-Sequenz des voranstehenden Anspruchs trägt.

19. Transformierte Wirtszelle, die die DNA-Sequenz gemäß Anspruch 17 oder den Expressionsvektor gemäß Anspruch 18 enthält.

20. Verfahren zum Herstellen der Lipase gemäß einem beliebigen der Ansprüche 1-12, wobei das Verfahren Kultivieren der transformierten Wirtszelle gemäß Anspruch 19 unter Bedingungen, die der Herstellung der Lipase förderlich sind, und Gewinnen der Lipase aus der sich ergebenden Brühe umfasst.

## Revendications

1. Lipase qui est un polypeptide ayant une séquence d'acides aminés qui :
a) présente au moins 90 % d'identité avec la lipase de type sauvage dérivée de la souche DSM 4109 de *Humicola lanuginosa* ;
b) comparativement à ladite lipase de type sauvage, comprend une substitution T231R ; et
i) comprend un acide aminé négatif en position E210 de ladite lipase de type sauvage ;
ii) comprend un acide aminé chargé négativement dans la région correspondant aux positions 90 à 101 de ladite lipase de type sauvage ; et
iii) comprend un acide aminé neutre ou négatif au niveau d'une position correspondant à N94 de ladite lipase de type sauvage et/ou a une charge électrique nette négative ou neutre dans la région correspondant aux positions 90 à 101 de ladite lipase de type sauvage.

2. Lipase selon la revendication 1, qui comprend une substitution d'un acide aminé électriquement neutre ou chargé négativement au niveau de l'une quelconque des positions 1 à 7, 10, 219 à 224 et 230 à 239 par R.

3. Lipase selon les revendications 1 ou 2, qui comprend une substitution de l'acide aminé S3, S224, P229, N233, D234 ou T244 par un acide aminé chargé positivement, de préférence R.

4. Lipase selon l'une quelconque des revendications précédentes, qui comprend une addition de peptide à l'extrémité C-terminale.

5. Lipase selon la revendication 4, où l'addition de peptide consiste en 1 à 5 acides aminés.

6. Lipase selon les revendications 4 ou 5, où l'addition de peptide consiste en des acides aminés électriquement neutres (de préférence hydrophobes), et est de manière préférée entre toutes PLG ou PG.

7. Lipase selon l'une quelconque des revendications 4 à 6, où l'addition de peptide consiste en des acides aminés neutres (de préférence hydrophobes) et C, et la lipase comprend en outre la substitution d'un acide aminé par C de manière à former un pont disulfure avec le C de l'addition de peptide.

8. Lipase selon l'une quelconque des revendications précédentes, qui comprend des acides aminés avec une charge électrique négative ou non changée dans au moins deux des positions N94, D96 et E99 de ladite lipase de type sauvage.

9. Lipase selon l'une quelconque des revendications précédentes, qui comprend une substitution G91A, N94D/E/K/R, D96E/I/L/N/S/W, E99N/Q/K/R/H, N101S, R209P/S ou Q249R/K/H.

10. Lipase selon l'une quelconque des revendications précédentes, qui comprend l'un des ensembles suivants de substitutions, éventuellement en combinaison avec Q249R/K/H et/ou K98X :
a) G91G/A/S/T + N94 (neutre ou négatif) + D96D/E/C/Y + E99E/D/C/Y,
b) G91G/A/S/T + N94 (neutre ou négatif) + D96D/E/C/Y + E99N + N101S,
c) G91G/A/S/T + N94R/K/H + D96D/E/C/Y + E99E/D/C/Y,
d) G91G/A/S/T + N94 (neutre ou négatif) + D96 (neutre ou positif) + E99E/D/C/Y, ou
e) G91G/A/S/T + N94 (neutre ou négatif) + D96D/E/C/Y + E99 (neutre ou positif).

11. Lipase selon l'une quelconque des revendications 1 à 10, qui comprend l'un des ensembles suivants de substitutions, éventuellement en combinaison avec R209 (neutre ou négatif) :
a) E99R/K/H + Q249R/K/H,
b) N94R/K/H + Q249R/K/H, ou
c) D96 (neutre ou positif) + Q249R/K/H.

12. Lipase selon l'une quelconque des revendications 1 à 11, qui comprend l'un des ensembles suivants de substitutions :
a) G91A + E99K + T231R + N233R + Q249R ;
b) E99N + N101S + T231R + N233R + Q249R + 270PGL ;
c) N33Q + E99N + N101S + T231R + N233R + Q249R + 270PGL ;
d) G91A + E99K + T231R + N233R + Q249R + 270PGL ;
e) N33Q + G91A + E99K + T231R + N233R + Q249R + 270PGL ;
f) E1A + G91A + E99K + T231R + N233R + Q249R + 270PGL ;
g) E1A + N33Q + G91A + E99K + T231R + N233R + Q249R + 270PGL ;
h) G91A + E99K + G255R + T231R + N233R + Q249R + 270PGL ;
i) N33Q + G91A + E99K + G255R + T231R + N233R + Q249R + 270PGL ;
j) G91A + E99K + T231R + N233R + T244R + Q249R + 270PGL ;
k) N33Q + G91A + E99K + T231R + N233R + T244R + Q249R + 270PGL ;
1) G91A + E99K + T231R + N233R + Q249R ;
m) G91A + E99K + T231R + N233R + Q249R + 270AGVF ;
n) G91A + E99K + T189G + T231R + N233R + Q249R ;
o) E1A + N33Q + G91A + E99K + T231R + N233R + Q249R + 270PGLPFKRV ;
p) N33Q + S83T + E87K + G91A + E99K + T231R + N233R + Q249R + 270PGLPFKRV ;
q) N33Q + G91A + E99K + T231R + N233R + Q249R + 270PGLPFKRV ;
r) N33Q + E99N + N101S + T231R + N233R + Q249R + 270PGLPFKRV ;
s) D62A + S83T + G91A + E99K + T231R + N233R + Q249R ;
t) E99N + N101S + T231R + N233R + Q249R ;
u) R84W + G91A + E99K + T231R + N233R + Q249R ;
v) G91A + E99K + T231R + N233R + Q249R + 270SPG ;
w) G91A + E99K + T231R + N233R + Q249R + 270VVVP ;
x) G91A + E99K + T231R + N233R + Q249R + 270LLASSGRGGHR ;
y) G91A + E99K + T231R + N233R + Q249R + 270VTT ;
z) G91A + E99K + T231R + N233R + Q249R + 270VLQ ;
aa) G91A + E99K + T231R + N233R + Q249R + 270TST ;
bb) G91A + E99K + T231R + N233R + Q249R + 270LRI ;
cc) V60G + D62E + G91A + E99K + T231R + N233R + Q249R ;
dd) G91A + D96W + E99K + T231R + N233R + G263Q + L264A + I265T + G266S + T267A + L269N + 270AGGFS ;
ee) G91A + D96W + E99K + G263Q + L264A + I265T + G266D + T267A + L269N 270AGGFSWRRYRSAESVDKRATMTDAELEKKLNSYVQMDKEYVKN NQARS.

13. Composition détergente comprenant un surfactant et la lipase selon l'une quelconque des revendications 1 à 12.

14. Composition détergente selon la revendication précédente, dans laquelle le surfactant comprend un surfactant anionique dans une quantité de plus de 70 % en poids du surfactant total.

15. Composition détergente selon la revendication 13, dans laquelle le surfactant comprend un surfactant anionique dans une quantité de 40 à 70 % en poids et un surfactant non ionique dans une quantité de 30 à 60 % en poids du surfactant total, et la lipase est la lipase selon les revendications 6 ou 7.

16. Composition détergente selon l'une quelconque des revendications 13 à 15, qui comprend 10 à 40 % en poids de surfactant, de préférence comprend 40 à 70 % d'adjuvant de détergence, et de préférence a un pH de 9 à 11 lorsqu'elle est dissoute dans de l'eau à 0,5 à 5 g/l.

17. Séquence d'ADN codant pour la lipase selon l'une quelconque des revendications 1 à 12.

18. Vecteur d'expression abritant la séquence d'ADN selon la revendication précédente.

19. Cellule hôte transformée contenant la séquence d'ADN selon la revendication 17 ou le vecteur d'expression selon la revendication 18.

20. Méthode de production de la lipase selon l'une quelconque des revendications 1 à 12, laquelle méthode comprend la culture de la cellule hôte transformée selon la revendication 19 dans des conditions conduisant à la production de la lipase et la récupération de la lipase à partir du bouillon résultant.
